# EUROPEAN PATENT APPLICATION

(11) **EP 3 949 970 A1**
(43) Date of publication of application: **09.02.2022**
(21) Application number: 20783154.6
(22) Date of filing: 24.03.2020
(51) Int. Cl.: A61K 31/569, A61P 35/00

(54) **COMBINED USE OF A-NOR-5? ANDROSTANE COMPOUND DRUG AND ANTICANCER DRUG**

(30) Priority: 04.04.2019 CN 201910272950
(71) Applicant: Shanghai Ao Qi Medical Technology Co., Ltd., Shanghai 200002 (CN)
(72) Inventor: CHEN, Yajun, Shanghai 200002 (CN); CHEN, Zhihua, Shanghai 200002 (CN); ZHENG, Yijun, Shanghai 200002 (CN)
(74) Representative: Rondano, Davide
(86) International application number: PCT/CN2020/080839
(87) International publication number: WO 2020/199973

(57) **Abstract**

A pharmaceutical composition comprising ACP (A-nor-5α androstane compound) as a first active ingredient and an anticancer drug as a second active ingredient. The composition has an antitumor function.

## Description

### TECHNICAL FIELD

The invention belongs to the technical field of medicine, in particular to treating tumor diseases through combined medication, specifically to combined medication of A-decarbonized-5α-androstane compound (ACP) and anti-tumor drugs to treat tumor diseases.

### BACKGROUND OF THE INVENTION

Malignant tumor has gradually replaced cardiovascular and cerebrovascular diseases as the number one killer in the world. The 2010 Cancer Report issued by the Fifth Asia-Pacific Cancer Prevention Organization Conference warned that the number of cancer patients in the world will show a rapid upward trend in the next 20 years. From 2008 to 2030, the number of new cancer patients worldwide will increase from 12.4 million annually to 26.4 million, of which patients in the Asia-Pacific region account for 60% of the total number of cancer patients in the world.

7.6 million people died of malignant tumors worldwide in 2007. In developed countries, the mortality rate of malignant tumors accounts for 21.6% of the total deaths. In China, the incidence of malignant tumors has increased significantly, with an annual incidence of about 2.6 million and 1.8 million deaths. The mortality rate has increased by 80% in 30 years. It has become the first cause of death for urban and rural residents in China. In China, lung cancer, gastric cancer, liver cancer and breast cancer are the main common malignant tumors. Traditional treatments for cancer include surgery, radiotherapy and chemotherapy. Most anti-tumor drugs currently used lack the effect of selectively inhibiting tumors. While inhibiting the growth and development of malignant tumors, they have obvious damage effects on normal cells of the body, especially the proliferating bone marrow cells, digestive tract mucosal epithelial cells, and germ cells. It has a certain toxic effect on the vital organs of the body: liver, kidney, heart, lung, nervous system, etc. There are even side effects such as bone marrow suppression, digestive tract reaction, alopecia, liver damage, cardiotoxicity, genitourinary system toxicity, carcinogenicity and teratogenicity, etc. Therefore, the research and development of anti-malignant tumor drugs with less toxic and side effects and good curative effect has been a hot issue.

In recent years, with the deepening of cell and molecular biology research, research and development of drugs for treating malignant tumors, especially screening anti-tumor drugs with high efficiency and low toxicity. A-decarbonized-5α-androstane compound (ACP) is a new compound independently developed and synthesized by Li Ruilin and others in 2000.

Up to now, there is no study on the combination of ACP in this field. Therefore, there is still a lack of a combination drug regimen that has good therapeutic effects and can significantly improve the tumor suppressive effect of ACP in this field.

### SUMMARY OF THE INVENTION

The purpose of the invention is to provide a combination drug regimen that has good therapeutic effects and can significantly improve the tumor inhibition effect of other anti-tumor drugs.

In the first aspect of the invention, it provides an anti-tumor pharmaceutical composition comprising:
(A) a therapeutically effective amount of a first active ingredient having an A-decarbonized-5α-androstane compound represented by the following formula I; 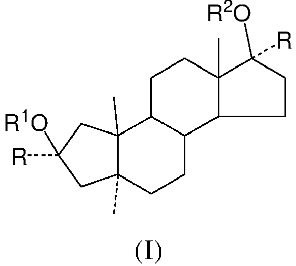 wherein R¹ and R² are each independently selected from H, substituted or unsubstituted-Ci-io alkyl, substituted or unsubstituted-C₃₋₈ cycloalkyl, substituted or unsubstituted benzene ring, substituted or unsubstituted benzoyl, substituted or unsubstituted-COCₙH₂ₙ₊₁, substituted or unsubstituted-COCᵣH₂ᵣCOOCₘH₂ₘ₊₁, or - COCₚH₂ₚCOO⁻W; wherein n, p, r and m are independently integers from 0 to 18, and W is H⁺, Na⁺, K⁺, NH₄⁺, 1/2 Ca^{2 +}, 1/2 Mg^{2 +}, 1/2 (AlOH)^{2 +}or 1/2 Zn^{2 +},
   R is selected from the group consisting of substituted or unsubstituted C₁₋₄alkynyl, cyano;
   the "substituted" means having one or more (e.g. 1-3) of the following substituting groups: hydroxyl, halogen, nitro, amino, amine, carboxyl;
   and (B) a therapeutically effective amount of a second active ingredient, and the second active ingredient is a second anti-tumor drug.

In another preferred embodiment, the A-decarbonized-5α-androstane compound is selected from the group consisting of:
2 α, 17 α-diethynyl-A-decarbonized-5 α-androstane-2 β, 17 β-diol;
2 α, 17 α-diethynyl-A-decarbonized-5 α-androstane-2 β, 17 β-dihydroxydiacetate;
2 α, 17 α-diethynyl-A-decarbonized-5 α-androstane-2 β, 17 β-dihydroxydipropionate;
2 α, 17 α-diethynyl-A-decarbonized-5 α-androstane-2 β, 17 β-dihydroxy-2 β-monosuccinate;
2 α, 17 α-diethynyl-A-decarbonized-5 α-androstane-2 β, 17 β-dihydroxybisuccinate;
2 α, 17 α-diethynyl-A-decarbonized-5 α-androstane-2 β, 17 β-dihydroxydibutyrate;
2 α, 17 α-dihydroxypropynyl-A-decarbonized-5 α-androstane-2 β, 17 β-diol;
2 α, 17 α-dicyano-A-decarbonized-5 α-androstane-2 β, 17 β-diol;
2 α, 17 α-diethynyl-A-decarbonized-5 α-androstane-2 β, 17 β-dihydroxyl ditrichloroacetate;
2 α, 17 α-diethynyl-A-decarbonized-5 α-androstane-2 β, 17 β-dihydroxy-2 β-propionate-17 β succinate;
2 α, 17 α-dipropynyl-A-decarbonized-5 α-androstane-2 β, 17 β-diol; or
2 α, 17 α-dipropynyl-A-decarbonized-5 α-androstane-2 β, 17 β-dihydroxydipropionate.

In another preferred embodiment, in the pharmaceutical composition, the mass ratio of the compound represented by Formula I to the second anti-tumor drug is 1: 10000 to 10000: 1; preferably 1: 1000 to 1000: 1.

In another preferred embodiment, the second anti-tumor drug is selected from the group consisting of chemical drug, biological agent, or a combination thereof.

In another preferred embodiment, the second anti-tumor drug is selected from the group consisting of cytotoxic anti-tumor drug, targeted inhibitor, antibody drug, antibody-drug conjugate (ADC), immune checkpoint inhibitor, or combinations thereof.

In another preferred embodiment, the targeted inhibitor is selected from sorafenib, sunitinib, lapatinib, pazopanib, axitinib, apatinib, nintedanib, bevacizumab, or combinations thereof.

In another preferred embodiment, the targeted inhibitor is selected from the group consisting of VEGF inhibitor, kinase inhibitor, EGFR inhibitor, Her2 inhibitor, or combinations thereof.

In another preferred embodiment, the targeted inhibitor is selected from VEGF inhibitor consisting of sorafenib, sunitinib, bevacizumab, or combinations thereof.

In another preferred embodiment, the immune checkpoint inhibitor comprises PD-1 monoclonal antibody, PD-L1 monoclonal antibody, or a combination thereof.

In another preferred embodiment, the cytotoxic anti-tumor drug is selected from the group consisting of alkaloid anti-tumor drug, anti-metabolic anti-tumor drug, antibiotic anti-tumor drug, alkylating anti-tumor drug, platinum anti-tumor drug, or combinations thereof.

In another preferred embodiment, the alkaloid anti-tumor drug is selected from paclitaxel, vinorelbine, vincristine, vindesine, hydroxycamptothecin, docetaxel, rrinotecan (CPT-11).

In another preferred embodiment, the anti-metabolic anti-tumor drug is selected from gemcitabine, cytarabine, tegafur, methotrexate, 5-fluorouracil (5-FU), calcium folinate, mercaptopurine, pemetrexed, pentostatin and calciumleucovorin (LV).

In another preferred embodiment, the antibiotic anti-tumor drug is selected from adriamycin (doxorubicin), actinomycin-D, actinomycin-C, mitomycin-C, daunorubicin hydrochloride, epirubicin hydrochloride, zorubicin hydrochloride, mitoxantrone hydrochloride and mitotan.

In another preferred embodiment, the alkylating anti-tumor drug is selected from cyclophosphamide, ifosfamide, busulfan, dacarbazine, formostine, prednimustine, carmostine, trastamide and melphalan.

In another preferred embodiment, the platinum anti-tumor drug is selected from cisplatin, carboplatin and oxaliplatin (OXA).

In another preferred embodiment, the second anti-tumor drug is a combination of irinotecan (CPT-11), 5-fluorouracil (5-FU), calciumleucovorin (LV), and bevacizumab.

In another preferred embodiment, the pharmaceutical composition further comprises (C) a pharmaceutically acceptable carrier.

In another preferred embodiment, the second anti-tumor drug is selected from the group consisting of sorafenib, bevacizumab, irinotecan (CPT-11), gemcitabine, 5-fluorouracil (5-FU), calciumleucovorin (LV), adriamycin (doxorubicin), cyclophosphamide, oxaliplatin (OXA), or combinations thereof.

In another preferred embodiment, the tumor disease is selected from the group consisting of pancreatic cancer, liver cancer, lung cancer, prostate cancer, kidney cancer, colon adenocarcinoma, colorectal cancer, melanoma, esophageal cancer, lymphoma, thoracic cancer, digestive tract cancer, colon cancer, breast cancer, uterine cancer, ovarian cancer, cholangiocarcinoma, testicular cancer, blood cancer, central nervous system cancer.

In another preferred embodiment, the tumor disease is pancreatic cancer.

In another preferred embodiment, the tumor disease is liver cancer.

In another preferred embodiment, the tumor disease is lung cancer.

In another preferred embodiment, the tumor disease is colon adenocarcinoma.

In the second aspect of the invention, it provides a combination of active ingredients comprising:
(A) a therapeutically effective amount of a first active ingredient having an A-decarbonized-5α-androstane compound represented by the following formula I; 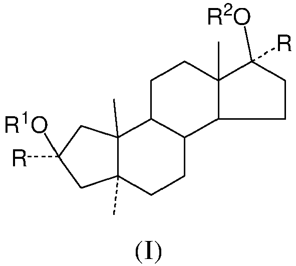 wherein R¹ and R² are each independently selected from H, substituted or unsubstituted-Ci-io alkyl, substituted or unsubstituted-C₃₋₈ cycloalkyl, substituted or unsubstituted benzene ring, substituted or unsubstituted benzoyl, substituted or unsubstituted-COCₙH₂ₙ₊₁, substituted or unsubstituted-COCᵣH₂ᵣCOOCₘH₂ₘ₊₁, or - COCₚH₂ₚCOO⁻W; wherein n, p, r and m are independently integers from 0 to 18, and W is H⁺, Na⁺, K⁺, NH₄⁺, 1/2 Ca^{2 +}, 1/2 Mg^{2 +}, 1/2 (AlOH)^{2 +} or 1/2 Zn^{2 +},
   R is selected from the group consisting of substituted or unsubstituted C₁₋₄ alkynyl, cyano;
   the "substituted" means having one or more (e.g. 1-3) of the following substituting groups: hydroxyl, halogen, nitro, amino, amine, carboxyl;
   and (B) a therapeutically effective amount of a second active ingredient, and the second active ingredient is a second anti-tumor drug.

In the third aspect of the invention, it provides a kit comprising: (a) the pharmaceutical composition described in the first aspect or the combination of active ingredients described in the second aspect; (b) container; and (c) an instruction manual or label indicating that the pharmaceutical composition or the combination is administered to a subject for the treatment of cancer.

In another preferred embodiment, the compound of Formula I and the second anti-tumor drug may be administered simultaneously, separately or sequentially.

It should be understood that in the present invention, any of the technical features specifically described above and below (such as in the Example) can be combined with each other, thereby constituting new or preferred technical solutions which will not redundantly be described one by one herein.

### DETAILED DESCRIPTION OF THE INVENTION

After long-term and in-depth research, the inventor discovered the existing anticancer drugs ACP and its analogues can be used in combination with a variety of anticancer drugs, and show synergistic drug effect. This combination is suitable for clinical treatment due to low toxicity and excellent therapeutic effect. The inventors have completed the present invention based on this discovery.

### ACP drugs

In this invention, that term "ACP drugs" refers to A-decarbonized-5α-androstane compound having the following structure: 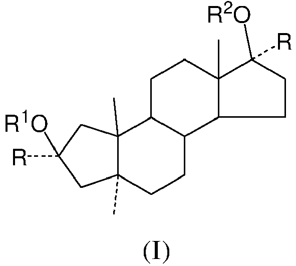 wherein, R¹ and R² are independently selected from H, substituted or unsubstituted-Ci-io alkyl, substituted or unsubstituted-C₃₋₈ cycloalkyl, substituted or unsubstituted benzene ring, substituted or unsubstituted benzoyl, substituted or unsubstituted-COCₙH₂ₙ₊₁, substituted or unsubstituted-COCᵣH₂ᵣCOOCₘH₂ₘ₊₁, or - COCₚH₂ₚCOO⁻W; wherein, n, p, r and m are each independently integer of 0~18, W is H⁺, Na⁺, K⁺, NH₄⁺, 1/2 Ca^{2 +}, 1/2 Mg^{2 +}, 1/2 (AlOH)^{2 +}, or 1/2 Zn^{2 +};
R is selected from the group consisting of substituted or unsubstituted C₁₋₄ alkynyl and cyano;
the "substituted" means having one or more (such as 1-3) following substituents: hydroxyl, halogen, nitro, amino, amine and carboxyl.
n, p, r and m are each independently integer of 0~18, which refers to that n, p, r and m can be independently selected from any integer between 0-18, i.e. 0, 1, 2, 3, 4, 5, 6, 7, 8, 9, 10, 11, 12, 13, 14, 15, 16, 17 and 18, preferably 0-6, more preferably 1-4. Preferably, the A-decarbonized-5α-androstane compound is:
   2 α, 17 α-diethynyl-A-decarbonized-5α-androstane-2 β, 17 β-diol (Ia);
   2 α, 17 α-diethynyl-A-decarbonized-5α-androstane-2 β, 17 β-dihydroxy diacetate (Ib);
   2 α, 17 α-diethynyl-A-decarbonized-5α-androstane-2 β, 17 β-dihydroxy dipropionate (Ic);
   2 α, 17 α-diethynyl-A-decarbonized-5α-androstane-2 β, 17 β-dihydroxyl-2 β-monosuccinate (Id);
   2 α,17 α-diethynyl-A-decarbonized-5α-androstane-2 β,17 β-dissuccinate (Ie);
   2 α,17 α-diethynyl-A-decarbonized-5α-androstane-2 β,17 β-dibutyrate (If);
   2 α, 17 α-dihydroxypropynyl-A-decarbonized-5α-androstane-2 β, 17 β-diol (Ig);
   2 α, 17 α-dicyano-A-decarbonized-5α-androstane-2 β,17 β-diol (Ih);
   2 α, 17 α-diethynyl-A-decarbonized-5α-androstane-2 β, 17 β-dihydroxyl ditrichloroacetate (Ii);
   2 α, 17 α-diethynyl-A-decarbonized-5α-androstane-2 β, 17 β-dihydroxyl-2 β-propionate-17 β-succinate (Ij);
   2 α,17 α-dipropynyl-A-decarbonized-5α-androstane-2 β,17 β-diol (Ik); or
   2 α,17 α-dipropynyl-A-decarbonized-5α-androstane-2 β,17 β-dihydroxydipropionate (Il).

### COMPOSITIONS, KITS, COMBINATIONS OF ACTIVE INGREDIENTS AND PHARMACEUTICAL COMPOSITIONS

The composition of the invention may be a pharmaceutical composition (drug), food or health product, the composition comprises:
(A) a therapeutic effective amount of ACP as the first active ingredient;
(B) a therapeutically effective amount of a second anti-tumor drug as the second active ingredient;
   and the mass ratio of the first active ingredient to the second active ingredient is 1: 10,000 to 10000:1; preferably, 1: 1000 to 1000:1.

In the pharmaceutical composition of the invention, the content range of the first active ingredient is 0.01% to 99.99%, based on the total weight of the composition. Preferably, 0.1% to 99.9%, more preferably, 20% to 99%. The content range of the second active ingredient is 0.01% to 99.99%, based on the total weight of the composition. Preferably, 1% to 99%, more preferably, 1% to 90%.

If necessary, the composition may also comprise acceptable carriers to pharmaceutics, bromatology, and health products. As used herein, the term ingredients "acceptable to pharmaceutics, bromatology, and health products" means substances suitable for applying to humans and / or animals without undue undesired side-reactions (such as toxicity, stimulation or allergy), that is, with reasonable benefit / risk ratio. As used herein, the term "effective amount" means an amount which may exert function or activity to human and / or animals and be acceptable for human and / or animal.

As use herein, the term "pharmaceutically acceptable carrier" refers to a carrier used for the administration of therapeutic agent, including various excipients and diluents. This term refers to such pharmaceutical carrier which themselves are not essential active components and without undue toxic after administration. Suitable carriers are well-known to those of ordinary skill in the art.

The pharmaceutics, food, health product compositions comprising the first active ingredient, the second active ingredient, or derivatives, metabolites thereof according to the present invention may be in various dosage forms suitable for oral administration, and may also be various topical formulations or other kinds of parenteral preparations. For example, the topical administration formulations of the present invention may also be further prepared into (including but not limited to): liniments, tinctures, oils, ointments, plasters, pastes, ironing agents, plaster, patch, plastics, films, gels, cataplasms, acupoint application formulations, sprays, aerosols, implants, emulsions and the like, by adding surfactants, penetration enhancers, preservatives, solvents, antioxidants, humectants, pH adjusting agents, colorants, perfumes and other auxiliary materials. For cancer, the preferred dosage forms include: various dosage forms for oral administration, implants, injections, etc.

The auxiliary materials added in the compositions of the present invention are commonly used auxiliary materials in the art, types, method for use and source of which are well-known to those skilled in the art.

The present invention also provided a combination of active ingredients, wherein the combination comprises the following ingredients, or is composed of the following ingredients:
(A) a first active ingredient, and the first active ingredient is ACP compound;
(B) a second active ingredient, and the second active ingredient is an anti-tumor drug.

In the combination, the mass ratio of the first active ingredient to the second active ingredient is 1: 10,000 to 10,000:1.

The invention also provides a kit comprising
(1) pharmaceutical composition of A-decarbonized-5α-androstane compound and a second anti-tumor drug or a combination of the two as the active ingredient; (2) container; and (3) instructions or labels indicating administration of the above combination of drugs to a subject for the treatment of cancer.

The composition, combination of active ingredients, pharmaceutical composition, kit, food and health products of the present invention all can be prepared by conventional methods and equipments.

### USE AND ADMINISTRATION MODE OF COMPOSITION, COMBINATION OF ACTIVE INGREDIENTS, PHARMACEUTICAL COMPOSITION, AND KIT

The present invention provides the use of the above composition, the active ingredient combination, the pharmaceutical composition in preparing drugs, health products or foods for inhibiting cancer cells, or in preparing health products or food for anti-tumor, or in preparing anti-tumor medicament.

The composition, kit, combination of active ingredients, and pharmaceutical composition can inhibit cancer cells such as pancreatic cancer, liver cancer, lung cancer, prostate cancer, kidney cancer, colon adenocarcinoma, colorectal cancer, melanoma, esophageal cancer, lymphoma, thoracic cancer, digestive tract cancer, colon cancer, breast cancer, uterine cancer, ovarian cancer, cholangiocarcinoma, testicular cancer, blood cancer and central nervous system cancer, etc. and can produce synergistic effect. Not limited to theory, the mechanisms of the composition, kit, combination of active ingredients, and pharmaceutical composition for inhibiting the growth and metastasis of cancer cells described herein are likely to be multi-layered and multi-targeted. It can inhibit cancer cells through several different mechanisms and ways.

The composition, combination of active ingredients, pharmaceutical composition and kit of the present invention may be used before, concurrently with or after the use of other cancer-treating active substances, cancer-specific surgery or cancer-specific radiation therapy, or in combination with gene therapy, or in combination with biological modulators.

In combination with the first preparation (containing a first active ingredient) and the second preparation (containing a second active ingredient) in the kit of the present invention, the first preparation and the second preparation can be administered simultaneously, separately or sequentially. The safe and effective daily dosage of the active ingredient in the first formulation typically is 0.1 mg - 2000 mg, preferably 1 mg - 500 mg, more preferably 1 mg - 300 mg, while the safe and effective daily dose of the active ingredient in the second formulation is generally 0.01 mg - 1500 mg, preferably 0.1 - 1500 mg, more preferably 1 mg - 1500 mg, more preferably 1 mg - 500mg. Administration modes include: when administered in combination, the first formulation can be administered orally, topically or by other parenteral routes, and the second formulation can be administered orally, topically or by other parenteral routes.

During the combined medication, the interaction between drugs, according to the effect of drugs when used together, can be sorted into adductive effect, synergy effect, antagonism effect, wherein synergy effect refers to that the effect of the drugs when used together is many times greater than that when used alone, adductive effect refers to that the effect of the drugs when used together equals to that when used alone, and antagonism effect refers to that the effect of the drugs when used together is inferior to that when used alone. In the present invention, it was firstly found that the first formula and the second formula have synergy effect.

A method for treating cancer is also provided in the present invention, which comprises the following steps: administering the composition, combination of active ingredients, pharmaceutical composition or kit of the present invention to a subject in need thereof, wherein the daily administration dose of the active ingredient is 1 mg to 10 mg. The subject is a mammal, preferably human.

In the inhibition of cancer cells or treatment of cancer, the administration mode of the present invention comprises successively administering the first active ingredient and the second active ingredient, or simultaneously administering the first active ingredient and the second active ingredient.

When administrating the composition, combination of active ingredients, pharmaceutical composition of the present invention, safe and effective amount of the composition, combination of active ingredients, pharmaceutical composition of the present invention is administered to a mammal, wherein the safe and effective daily amount of the first active ingredient is typically at least 0.1 mg, and in most cases, less than 2000 mg. Preferably, the amount is 1 mg - 500 mg. The safe and effective amount of the second active ingredient is typically at least about 0.01 mg, and in most cases, less than 1500 mg. Preferably, the amount is 0.1 mg to 1500 mg. (Wherein, the safe and effective amount of the first active ingredient will usually less than about 2000 mg / kg of body weight. Preferably, the amount is about 100 µg/kg of body weight to about 1000 mg/ kg of body weight; the safe and effective amount of the second active ingredient is usually less than about 2000 mg/kg of body weight. Preferably, the amount is about 10 µg/kg of body weight to about 1000 mg/ kg of body weight). Of course, the particular dose should also depend on various factors, such as the route of administration, healthy status of a patient, which are all well within the skills of an experienced physician. There is no specific requirement on the interval of administration when successively administering the first active ingredient and the second active ingredient. The compositions, active ingredient combinations, pharmaceutical compositions and the first and second preparations in the kit are administered simultaneously or successively in the same or different routes, respectively, including but not limited to oral administration, injection administration, intratumoral administration, implantation administration, intraluminal administration, anal administration, transdermal administration, internal and external application;

Preferred injections include intravenous injection, intramuscular injection, subcutaneous injection and intracavitary injection.

Efficacy evaluation index:

| Serial number | Main evaluation criteria | Meaning |
|---|---|---|
| 1 | Cell morphology(Pathology) | The higher the degree of atypia, the greater the proportion of moderate and severe atypia cells, and the higher the malignant degree of tumor |
| 2 | Proportion of tumor components(Pathology) | Judging the proportion of tumor components in tumor tissue(containing for example, fat, connective tissue, etc) |
| 3 | Proportion of tumor necrosis components TCNR (Pathology) | According to the proportion of necrosis components in tumor tissue, the killing effect of different treatment groups on tumor cells was judged |
| 4 | Tumor Ki67 (Immunohistochemistry) | The proliferation of tumor tissue per unit volume under different treatment schemes, at the same time, it is helpful to accurately judge the prognosis of patients |

Ki-67 is a protein that exists in the nucleus of cells and Ki was named because the city in which it was discovered was Kiel, Germany, 67 came from the experimental number. Ki-67 is very active in the process of cell proliferation, but it disappears when the proliferation stops, so it can be used as a sign of cell proliferation state to judge the malignant degree of various malignant tumors.

The higher the positive expression rate of Ki-67, the stronger the proliferation activity of tumor cells, the higher the malignant degree, and the higher the risk of recurrence and metastasis of patients. Ki67 is examined in almost all cancers (including lung cancer, breast cancer, prostate cancer, cervical cancer, colorectal cancer, bladder cancer, etc) at the time of pathology. The higher the Ki67 value, the worse it is. However, everything has two sides. Tumors with a high Ki67 positive rate are often more sensitive to chemotherapy, and the chemotherapy effect will be better.

In specific animal experiments, the present invention applies SuperPDTX mouse models established by implanting tumor tissues from patients into highly immune deficiency model NCG (NOD-Prkdcem26Il2rgem26Nju), tumor inhibitory effect of the combination of ACP and gemcitabine, oxaliplatin or sorafenib was better than that of single administration, showing synergistic effect. In the treatment of a patient with liver metastasis from colon adenocarcinoma, FOLFIRI combined with bevacizumab along with oral ACP, the liver metastasis was obviously reduced before and after treatment. ACP combined with low-dose cyclophosphamide or adriamycin had an additive effect on the growth inhibition of human tumor liver cancer QGY-7703, pancreatic cancer PANC-1, lung cancer A549, showing certain combined drug effect.

The present invention will be further illustrated below with reference to the specific examples. It should be understood that these examples are only to illustrate the invention but not to limit the scope of the invention. In the following examples, the test methods without specific conditions are usually in accordance with conventional conditions or in accordance with the conditions recommended by the manufacturer. Unless otherwise stated, percentage and parts are calculated by weight. Unless otherwise stated, all technical and scientific terms used in this specification have the same meanings as those generally understood by the skilled in the art.

Example 1 Efficacy of ACP combined with gemcitabine in the treatment of pancreatic cancer in SuperPDTX mice model

### 1. Experimental design

The experimental design of pharmacodynamics was shown in the Table 1

**Table 1.**

| **Group number** | **Number of animals** | **Treatment** | **Single dose** | **Route of administration** | **Administration arrangement** |
|---|---|---|---|---|---|
| 1 | 6 | Blank control group | 0mg/kg | p.o. | Qd^{∗}7 |
| 2 | 6 | ACP group | 5mg/kg | p.o. | Bid^{∗}7 |
| 3 | 6 | Gemcitabine group | 30mg/kg | i.p. | BIW |
| 4 | 6 | ACP | 5mg/kg | p.o. | Bid^{∗}7 |
| | | Gemcitabine | 30mg/kg | i.p. | BIW |

### 2. Materials

Mice: NCG (NOD-Prkdcem26Il2rgem26Nju), Institute of Biomedical Research, Nanjing University

Pancreatic cancer tissue mass were the P2 generation samples of cryopreserved collected from mice after inoculation of the samples from patients with pancreatic cancer, and the samples used in this experiment were P3 generation samples after passage.

### 3. Experimental method

Pancreatic cancer samples were inoculated in the tested mice, and the size of inoculated tissue was 1^{∗}1^{∗}4mm³. On the day of inoculation, mice were randomly divided into 4 experimental groups with 6 mice in each group and 4 mice in each cage. The day of grouping was defined as Day 0. The mice were administered according to the experimental protocol for 7 days, and sampled within 24 hours after the end of administration. 24h after the last administration, the experiment was ended, the mice were sacrificed, the tumor was removed, the surrounding connective tissue and muscle tissue were removed under the microscope, and stored in 10% formaldehyde.

### 4. Results

Sample test results were shown in Table 2.

**Table 2**

| Serial number | Detection scheme | Tumor component Mean | Heterotypi c degree | Proportion of tumor necrosis TCNR Mean | Tumor Ki67 Mean | Compared with the blank control group (ki67) P Value |
|---|---|---|---|---|---|---|
| 1 | Blank control group | 88.33 ±4.08 | Moderate | 0.83 ±2.04 | 45.00 ±5.48 | -- |
| 2 | ACP group | 83.33 ±5.16 | Moderate | 1.67 ±4.08 | 41.67 ±11.69 | 0.417 |
| 3 | Gemcitabine | 55.00 | Moderate | 0.00 | 23.33 | ^{∗∗}0.00 |
| | group | ±18.71 | | ±0.00 | ±5.16 | |
| 4 | ACP +Gemcitabine group | 51.67 ±26.39 | Moderate | 8.33 ±16.02 | ##ΔΔ10. 83 ±4.92 | ^{∗∗}0.00 |

Compared with the blank control group, ^{∗}p<0.05, ^{∗∗}p<0.01. Compared with the single ACP Group, # p<0.05, ##p<0.01. Compared the combination group with gemcitabine group, ΔΔ p<0.01

Compared with blank control group, the expression of Ki67 in ACP group showed a downward trend, but no statistical difference was observed. ACP alone had a certain inhibitory effect on tumor proliferation, and the combination of ACP and Gemcitabine had a significantly better inhibitory effect on tumor proliferation than ACP alone (p <0.01) and Gemcitabine alone (p <0.01), and the killing effect was also significantly better than the single drug group. The combination of ACP and Gemcitabine had a significant synergistic effect.

### Example 2 Efficacy of ACP combined with oxaliplatin or sorafenib in the treatment of liver cancer in SuperPDTX mice model

### 1. Experimental design

**Table 3**

| **Group number** | **Number of animals** | **Treatment** | **Single dose** | **Route of administration** | **Administration arrangement** |
|---|---|---|---|---|---|
| 1 | 6 | Negative control group | 0mg/kg | p.o. | Qd^{∗}7 |
| 2 | 6 | ACP group | 5mg/kg | p.o. | Bid^{∗}7 |
| 3 | 6 | Sorafenib | 60mg/kg | p.o. | Qd^{∗}7 |
| 4 | 6 | Oxaliplatin | 6mg/kg | I.p. | Biw^{∗}1 |
| 5 | 6 | ACP | 5mg/kg | p.o. | Bid^{∗}7 |
| | | Oxaliplatin | 6mg/kg | I.p. | Biw^{∗}1 |
| 6 | 6 | ACP | 5mg/kg | p.o. | Bid^{∗}7 |
| | | Sorafenib | 60mg/kg | p.o. | Qd^{∗}7 |

### 2. Materials

Mice: NCG (NOD-Prkdcem26Il2rgem26Nju), Institute of Biomedical Research, Nanjing University

### 3. Experimental method

### 3.1 Tumor inoculation and grouping

Liver cancer tissue mass were the P1 generation samples of cryopreserved collected from mice after inoculation of the samples from patients with liver cancer, and the samples used in this experiment were P2 generation samples after passage. Liver cancer samples were inoculated in the tested mice, and the size of inoculated tissue was 1^{∗}1^{∗}4mm³. On the day of inoculation, mice were randomly divided into 6 experimental groups with 6 mice in each group and 4 mice in each cage. The day of grouping was defined as Day 0. The mice were administered according to the experimental protocol for 7 days, and sampled within 24 hours after the end of administration. 24h after the last administration, the experiment was ended, the mice were sacrificed, the tumor was removed, the surrounding connective tissue and muscle tissue were removed under the microscope, and stored in 10% formaldehyde.

### 4. Results

The results were shown in Table 4.

**Table 4**

| Serial number | Detection scheme | Tumor component Mean | Heterotypic Degree | Proportion of tumor necrosis TCNR Mean |
|---|---|---|---|---|
| 1 | Blank control group | 48.33±19.4% | Severe | 16.67±11.69% |
| 2 | ACP group | 54.17±24.6% | Severe | 23.33±13.67% |
| 3 | Sorafenib group | 58.33±24.8% | Severe | 18.33±7.52% |
| 4 | Oxaliplatin group | 53.33±15.1% | Severe | 16.67±8.76% |
| 5 | ACP +Oxaliplatin group | 51.67±18.3% | Severe | ^{∗}38.33±24.01% |
| 6 | ACP +Sorafenib group | 51.67±19.4% | Severe | ^{∗}40.00±12.65% |

| | | | | |
|---|---|---|---|---|
| ^{∗}Compared with the blank control group, p<0.05 | | | | |

The experimental results show that, compared with the Blank control group, the killing effect on tumor of ACP +Oxaliplatin group and ACP + Sorafenib group were obviously improved (p<0.05), showing a certain synergistic effect.

### Example 3 In vivo test model study of combination of ACP and Cyclophosphamide or Adriamycin for human tumor liver cancer QGY-7703, pancreatic cancer PANC-1, lung cancer A549

### 1. Administration regimen

**ACP Administration Group**: oral doses were 5, 2.5 and 1.25mg/ kg; oral administration twice a day (with an interval of about 6 hours) for 14 days.

**ACP +Adriamycin or Cyclophosphamide Administration Group**: oral doses of ACP were 5, 2.5 and 1.25mg/ kg; oral administration twice a day (with an interval of about 6 hours) for 14 days. Adriamycin 1mg/kg or Cyclophosphamide 15mg/kg were administered intraperitoneally 10 min after the first ACP administration for 7 consecutive days.

**Positive Control Group**: Adriamycin 2mg/kg/ time or cyclophosphamide 30mg/kg/ time were administered intraperitoneally once a day for 7 days.

**Negative Control Group**: the same volume of solvent as the experimental group was administered, and the administration scheme was the same as that of the experimental group.

### 2. Establishment of in vivo test model

Human tumor cell lines cryopstored in liquid nitrogen were cultured at 37°C and 5% CO₂ after resuscitation. After subculture, the cells in logarithmic growth period were taken and prepared into cell suspension of about (1-2) × 10⁷ Cell/ml, and inoculated subcutaneously in the right axilla of nude mice 0.2 ml/mice. Under aseptic conditions, tumor tissues of the second or later generations of human tumors liver cancer QGY-7703, pancreatic cancer PANC-1, and lung cancer A549 xenograft models with vigorous growth in vivo, were taken and cut into uniform pieces with the size of 1-2 mm3, and one piece was subcutaneously inoculated in the right axilla of each naked mouse with trocar. The mouse were randomly divided into groups till the tumors inoculated grow to more than 100 mm³ and administered according to the experimental design scheme.

### 3. Detection index of the test in vivo

After administration, the tumor size was dynamically observed and tested, and the body weight of tumor-bearing mice was weighed. The short diameter (a) and long diameter (b) of the tumor of the naked mouse were measured with calipers every 3 days, and the tumor volume was calculated according to the formula of (a² × b)/2. The relative tumor volume (RTV) was calculated from the measured and calculated tumor volume, RTV = Vₜ/V₀. Wherein, Vo is the tumor volume at random grouping (i.e., do), and Vₜ is the tumor volume at each measurement (i.e., dₙ). The animals in each group were sacrificed at about three weeks, and the tumor volume was measured, dissected and weighed. The relative tumor proliferation rate and tumor inhibition rate were calculated according to the following formula:

### 4. The results were as follows

Table 5 Antitumor effect of ACP samples combined with cyclophosphamide on human liver cancer QGY-7703 xenograft transplantation model in nude mice

| (Counted by tumor weight) | | | | | |
|---|---|---|---|---|---|
| Sample group | Dose | Administrat ion regimen | Number of animals Began/Ende d | Tumor weight (gram) *x̅* ± SD | Tumor inhibition rate% |
| ACP sample | 5mg/kg | ig×14Bid | 6/6 | 0.685±0.13^{∗∗} | 57.19 |
| ACP sample | 2.5mg/kg | ig×14 Bid | 6/6 | 0.788±0.12^{∗∗} | 50.75 |
| ACP sample | 1.25mg/k g | ig×14 Bid | 6/6 | 1.000±0.04^{∗∗} | 37.50 |
| ACP+cyclopho sphamide | (5mg+15 mg)/kg | ig×14 Bid +ip×7qd | 6/6 | 0.488±0.03^{∗∗} | 69.50 |
| ACP+cyclopho sphamide | (2.5mg+1 5mg)/kg | ig×14 Bid +ip×7qd | 6/6 | 0.462±0.04^{∗∗} | 71.13 |
| ACP+cyclopho | (1.25mg+ | ig×14 Bid | 6/6 | 0.518±0.14^{∗∗} | 67.63 |

| | | | | | |
|---|---|---|---|---|---|
| sphamide | 15mg)/kg | +ip×7qd | | | |
| Cyclophospha mide | 15mg/kg | ip×7qd | 6/6 | 1.010±0.19^{∗∗} | 36.88 |
| Cyclophospha mide | 30mg/kg | ip×7qd | 6/6 | 0.197±0.04^{∗∗} | 87.69 |
| Solvent Control | Correspo nding solvent | ig×14 Bid | 12/12 | 1.600±0.36 | |

Compared with the solvent control group:^{∗}*P*<0.05, ^{∗∗}*P*<0.01 Table 6 Antitumor effect of ACP sample combined with adriamycin on xenograft transplantation model of human pancreatic cancer PANC-1 in nude mice

| (Counted by tumor weight) | | | | | |
|---|---|---|---|---|---|
| Sample Group | Dose | Administrat ion regimen | Number of animals Began/Ended | Tumor weight (gram) *x̅* ± SD | Tumor Inhibition rate% |
| ACP sample | 5mg/kg | ig×14Bid | 6/6 | 0.548±0.10^{∗∗} | 46.27 |
| ACP sample | 2.5mg/kg | ig×14 Bid | 6/6 | 0.585±0.09^{∗∗} | 42.65 |
| ACP sample | 1.25mg/kg | ig×14 Bid | 6/6 | 0.653±0.13^{∗∗} | 35.98 |
| ACP + adriamycin | (5mg+1mg) /kg | ig×14 Bid +ip×7qd | 6/6 | 0.435±0.11^{∗∗} | 57.35 |
| ACP + adriamycin | (2.5mg+1m g)/kg | ig×14 Bid +ip×7qd | 6/6 | 0.458±0.07^{∗∗} | 55.10 |
| ACP + adriamycin | (1.25mg+1 mg)/kg | ig×14 Bid +ip×7qd | 6/6 | 0.467±0.09^{∗∗} | 54.22 |
| adriamycin | 1mg/kg | ip×7qd | 6/6 | 0.668±0.09^{∗∗} | 34.51 |
| adriamycin | 2mg/kg | ip×7qd | 6/6 | 0.265±0.06^{∗∗} | 74.02 |
| solvent control | Correspondi ng solvent | ig×14 Bid | 12/12 | 1.020±0.19 | |

Compared with the solvent control group:^{∗}*P*<0.05, ^{∗∗}*P*<0.01

Table 7 Antitumor effect of ACP sample combined with adriamycin on xenograft transplantation model of human lung cancer A549 in nude mice

| (Counted by tumor weight) | | | | | |
|---|---|---|---|---|---|
| Sample group | Dose | Administration regimen | Number of animals Began/Ended | Tumor weight (gram) *x̅* ± SD | Tumor Inhibition rate% |
| ACP sample | 5mg/kg | ig×14Bid | 6/6 | 0.683±0.14^{∗∗} | 53.22 |
| ACP sample | 2.5mg/kg | ig×14 Bid | 6/6 | 0.800±0.08^{∗∗} | 45.21 |
| ACP sample | 1.25mg/kg | ig×14 Bid | 6/6 | 0.888±0.14^{∗∗} | 32.33 |
| ACP + | (5mg+1mg)/kg | ig×14 Bid | 6/6 | 0.437±0.11^{∗∗} | 70.07 |
| adriamycin | | +ip×7qd | | | |
| ACP + | (2.5mg+1mg)/kg | ig×14 Bid | 6/6 | 0.468±0.12^{∗∗} | 67.95 |
| adriamycin | | +ip×7qd | | | |
| ACP + | (1.25mg+1mg)/kg | ig×14 Bid | 6/6 | 0.472±0.13^{∗∗} | 67.69 |
| adriamycin | | +ip×7qd | | | |
| adriamycin | 1mg/kg | ip×7qd | 6/6 | 0.843±0.08^{∗∗} | 42.26 |
| adriamycin | 2mg/kg | ip×7qd | 6/6 | 0.198±0.04^{∗∗} | 86.30 |
| solvent | Corresponding | ig×14 Bid | 12/12 | 1.460±0.31 | |
| control | solvent | | | | |

Compared with the solvent control group:^{∗}*P*<0.05, ^{∗∗}*P*<0.01

The results in Tables 5-7 above showed that ACP combined with low-dose cyclophosphamide or adriamycin had an additive effect on the growth inhibition of the human tumor liver cancer QGY-7703, pancreatic cancer PANC-1, lung cancer A549, showing certain combined drug effect.

Statistical data showed that the killing effect of combined drugs on tumor was obviously improved (p<0.05), showing a certain combined drug effect, especially when the dosage of ACP was low (for example about 1.25 mg/kg), the synergistic effect was more significant.

### Example 4 Effect of ACP used in combination on patient with liver metastasis from colon adenocarcinoma

### 1. Experimental design

Combination chemotherapy: Chemotherapy performed on FOLFIRI Methods: Irinotecan (CPT-11) +5-Fluorouracil (5-FU) +Calcium tetrahydrofolate (LV): started from November 27, 2014

**Targeted therapy:** Bevacizumab, at the same time as FOLFIRI.

**Along with oral ACP treatment:** started from November 27, 2014, one tablet each time, q.i.d. The chemotherapy was performed for 5 cycles and ACP was continued.

Results of combination chemotherapy: the liver metastasis was significantly reduced before and after treatment.

**Table 8**

| **Pre-treatment examination** | **Results** |
|---|---|
| 20141010CT | A space occupying lesion was found in the transverse colon of the patient, with a length larger than 6cm. The largest mass that could be detected was 4.2 x4cm. Diffuse liver metastases, the larger ones were 3.1 x3.1cm, 2.6 x2.6cm and 2.4 x2.5cm, with numerous small lumps below 1cm. The results showed that the colon lumps might be malignant. Colon lumps was malignant. |
| 20141125MR | There were multiple abnormal signal foci of different sizes in the liver, and the most was about 55mm. Conclusion: there were multiple liver metastases. |
| 20141126PET | The wall of splenic curvature of transverse colon was thickened, the lesser curvature of stomach was involved locally, and the radioactivity was abnormally concentrated. There were multiple slightly low-density foci in the liver, with clear boundaries, and the larger one was in the right lobe 9.1 x4.9cm. In the left lobe, there were multiple cystic low-densitv foci with a diameter of |
| | about 1.0 cm. |
| **Post- treatment examination** | **Results of CT** |
| 2015.1.12CTT | Compare with the results on November 25, multiple lesions were observed in the liver, some of which were smaller than before, and the largest one was about 27x29mm at present. |
| 2015.2.6CT | Compared with the results on January 12, multiple low-density nodules were observed in the liver, the largest was 2.6x2.6cm, and the lesions were similar to the previous ones. |
| 2015.3.19MR | Compared with the previous film, the lesions on the left transverse colon and multiple metastases in the liver were reduced to 2.1×2.0cm. |
| 2015.4.16MR | Compared with the previous film, the left wall of transverse colon was thickened with enhancement similar to the previous one, and there were multiple nodules in liver, and the larger was 2.1 x2.0cm, similar to previous one. |
| 2015.5.6MR | Compared with the previous film, the transverse colon wall thickened was less than the previous film, and the liver had multiple nodules, the largest was 2.1 x2.2 cm. The distribution and size were similar to those before. |

The results of tumor marker CA125 and CEA after combination chemotherapy were shown in Table 9:

**Table 9**

| | CA125 (U/ml) Normal value < 35 | CEA (ng/ml) Normal value < 5.20 | CA724 (U/ml) Normal value < 6.9 |
|---|---|---|---|
| November 27, 2014, prior-treatment | 37.38 | 7.35 | 47.86 |
| December 11, 2014, | 33.09 | 2.32 | NA |
| post-treatment | | | |
| December 24, 2014, post-treatment | 29.03 | 2.46 | 16.26 |
| January 12, 2015, post-treatment | 28.94 | 2.21 | NA |
| March 17, 2015, post-treatment | 16.32 | 2.72 | 16.32 |
| April 17, 2015, post-treatment | 34.97 | 1.49 | 25.69 |
| May 6,2015, post-treatment | 30.34 | 1.84 | 51.22 |

Chemotherapy was performed by FOLFIRI Methods: Postoperative adjuvant treatment lasted for 2 courses, and ACP continued to be treated until recovery.

The results showed that the patients without surgical indications obtained a chance for radical surgery after administration of the combination of ACP and a second antineoplastic agent. After the surgery, the patient continued to take ACP and is in good condition and has no recurrence at present.

All literatures mentioned in the present application are incorporated by reference herein, as though individually incorporated by reference. Additionally, it should be understood that after reading the above teaching, many variations and modifications may be made by the skilled in the art, and these equivalents also fall within the scope as defined by the appended claims.

## Claims

1. An anti-tumor pharmaceutical composition comprising:
(A) a therapeutically effective amount of a first active ingredient having an A-decarbonized-5α-androstane compound represented by the following formula I; 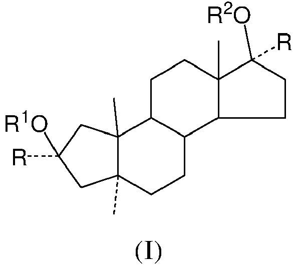 wherein R¹ and R² are each independently selected from H, substituted or unsubstituted-Ci-ioalkyl, substituted or unsubstituted-C₃₋₈cycloalkyl, substituted or unsubstituted benzene ring, substituted or unsubstituted benzoyl, substituted or unsubstituted-COCₙH₂ₙ₊₁, substituted or unsubstituted-COCᵣH₂ᵣCOOCₘH₂ₘ₊₁, or - COCₚH₂ₚCOO⁻W; wherein n, p, r and m are independently integers from 0 to 18, and W is H⁺, Na⁺, K⁺, NH₄⁺, 1/2 Ca^{2 +}, 1/2 Mg^{2 +}, 1/2 (AlOH)^{2 +}or 1/2 Zn^{2 +},
R is selected from the group consisting of substituted or unsubstituted C₁₋₄alkynyl, cyano;
the "substituted" means having one or more (e.g. 1-3) of the following substituents: hydroxyl, halogen, nitro, amino, amine, carboxyl;
and (B) a therapeutically effective amount of a second active ingredient, and the second active ingredient is a second anti-tumor drug.

2. The anti-tumor pharmaceutical composition of claim 1, wherein the A-decarbonized-5α-androstane compound is selected from the group consisting of:
2 α, 17 α-diethynyl-A-decarbonized-5 α-androstane-2 β, 17 β-diol;
2 α, 17 α-diethynyl-A-decarbonized-5 α-androstane-2 β, 17 β-dihydroxydiacetate;
2 α, 17 α-diethynyl-A-decarbonized-5 α-androstane-2 β, 17 β-dihydroxydipropionate;
2 α, 17 α-diethynyl-A-decarbonized-5 α-androstane-2 β, 17 β-dihydroxy-2 β-monosuccinate;
2 α, 17 α-diethynyl-A-decarbonized-5 α-androstane-2 β, 17 β-dihydroxybisuccinate;
2 α, 17 α-diethynyl-A-decarbonized-5 α-androstane-2 β, 17 β-dihydroxydibutyrate;
2 α, 17 α-dihydroxypropynyl-A-decarbonized-5 α-androstane-2 β, 17 β-diol;
2 α, 17 α-dicyano-A-decarbonized-5 α-androstane-2 β, 17 β-diol;
2 α, 17 α-diethynyl-A-decarbonized-5 α-androstane-2 β, 17 β-dihydroxyl ditrichloroacetate;
2 α, 17 α-diethynyl-A-decarbonized-5 α-androstane-2 β, 17 β-dihydroxy-2 β-propionate-17 β succinate;
2 α, 17 α-dipropynyl-A-decarbonized-5 α-androstane-2 β, 17 β-diol;or
2 α, 17 α-dipropynyl-A-decarbonized-5 α-androstane-2 β, 17 β-dihydroxydipropionate.

3. The anti-tumor pharmaceutical composition of claim 1, wherein the second anti-tumor drug is selected from the group consisting of chemical drug, biological agent, or a combination thereof.

4. The anti-tumor pharmaceutical composition of claim 3, wherein the second anti-tumor drug is selected from the group consisting of cytotoxic anti-tumor drug, targeted inhibitor, antibody drug, antibody-drug conjugate (ADC), immune checkpoint inhibitor, or combinations thereof.

5. The anti-tumor pharmaceutical composition of claim 4, wherein the cytotoxic anti-tumor drug is selected from the group consisting of alkaloid anti-tumor drug, anti-metabolic anti-tumor drug, antibiotic anti-tumor drug, alkylating anti-tumor drug, platinum anti-tumor drug, or combinations thereof.

6. The anti-tumor pharmaceutical composition of any one of claims 1-5, wherein the pharmaceutical composition further comprises (C) a pharmaceutically acceptable carrier.

7. The anti-tumor pharmaceutical composition of any one of claims 1-5, wherein the second anti-tumor drug is selected from the group consisting of sorafenib, bevacizumab, irinotecan (CPT-11), gemcitabine, 5-fluorouracil (5-FU), calciumleucovorin (LV), adriamycin (doxorubicin), cyclophosphamide, oxaliplatin (OXA), or combinations thereof.

8. The anti-tumor pharmaceutical composition of claim 1, wherein the tumor disease is selected from the group consisting of pancreatic cancer, liver cancer, lung cancer, prostate cancer, kidney cancer, colon adenocarcinoma, colorectal cancer, melanoma, esophageal cancer, lymphoma, thoracic cancer, digestive tract cancer, colon cancer, breast cancer, uterine cancer, ovarian cancer, cholangiocarcinoma, testicular cancer, blood cancer, and central nervous system cancer.

9. A combination of active ingredients comprising:
(A) a therapeutically effective amount of a first active ingredient having an A-decarbonized-5α-androstane compound represented by the following formula I; 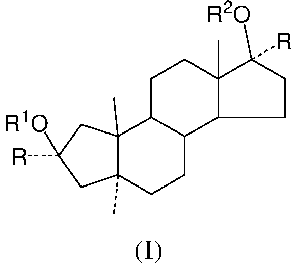 wherein R¹ and R² are each independently selected from H, substituted or unsubstituted-Ci-io alkyl, substituted or unsubstituted-C₃₋₈ cycloalkyl, substituted or unsubstituted benzene ring, substituted or unsubstituted benzoyl, substituted or unsubstituted-COCₙH₂ₙ₊₁, substituted or unsubstituted-COCᵣH₂ᵣCOOCₘH₂ₘ₊₁, or - COCₚH₂ₚCOO⁻W; wherein n, p, r and m are independently integers from 0 to 18, and W is H⁺, Na⁺, K⁺, NH₄⁺, 1/2 Ca^{2 +}, 1/2 Mg^{2 +}, 1/2 (AlOH)^{2 +}or 1/2 Zn^{2 +},
R is a group selected from the group consisting of substituted or unsubstituted C₁₋₄alkynyl, cyano;
the "substituted" means having one or more (e.g. 1-3) of the following substituting groups: hydroxyl, halogen, nitro, amino, amine, carboxyl;
and (B) a therapeutically effective amount of a second active ingredient, and the second active ingredient is a second anti-tumor drug.

10. A kit comprising (a) the pharmaceutical composition of claim 1 or the combination of active ingredients of claim 9; (b) container; and (c) an instruction manual or label indicating that the pharmaceutical composition or the combination is administered to a subject for the treatment of cancer.
